(19) Europäisches Patentamt
European Patent Office
Office européen des brevets

(11) Veröffentlichungsnummer : **0 041 134**
**B1**

# (12) EUROPÄISCHE PATENTSCHRIFT

(45) Veröffentlichungstag der Patentschrift : **13.06.84**

(51) Int. Cl.³ : **C 07 C139/14**, C 07 C143/30, C 07 C143/55, C 07 D221/10

(21) Anmeldenummer : **81103378.6**

(22) Anmeldetag : **05.05.81**

## (54) Verfahren zur Abtrennung wasserlöslicher Salze von aromatischen Sulfonsäuren aus Sulfiergemischen.

(30) Priorität : **30.05.80 DE 3020526**

(43) Veröffentlichungstag der Anmeldung : **09.12.81 Patentblatt 81/49**

(45) Bekanntmachung des Hinweises auf die Patenterteilung : **13.06.84 Patentblatt 84/24**

(84) Benannte Vertragsstaaten : **AT BE CH DE FR GB IT LI NL SE**

(56) Entgegenhaltungen :
**DE-C- 886 144**
**US-A- 2 800 503**
**US-A- 3 551 460**
**US-A- 3 719 703**
**Patent Abstracts of Japan Band 1, Nr. 53, 23. Mai 1977 Seite 323C77**

(73) Patentinhaber : **BASF Aktiengesellschaft**
**Carl-Bosch-Strasse 38**
**D-6700 Ludwigshafen (DE)**

(72) Erfinder : **Brunnmueller, Fritz, Dr.**
**Leopoldstrasse 19**
**D-6700 Ludwigshafen (DE)**
Erfinder : **Boehm, Walter, Dr.**
**Mittlerer Waldweg 11**
**D-6719 Kirchheim (DE)**
Erfinder : **Weberndoerfer, Volkmar, Dr.**
**Feldbergstrasse 48**
**D-6800 Mannheim (DE)**

**EP 0 041 134 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein vereinfachtes und umweltfreundliches Verfahren zur Gewinnung sulfatarmer wasserlöslicher Sulfonate aus Sulfiergemischen. Bei der Sulfierung von aromatischen Kohlenwasserstoffen mit Schwefelsäure oder Oleum kann die Reaktion meist nicht so geleitet werden, daß alle Schwefelsäure verbraucht wird, da sie durch Verdünnung mit dem Reaktionswasser ihre Reaktivität mehr oder minder stark einbüßt. Die unmittelbare Neutralisation des Sulfiergemisches ergäbe also stark Sulfathaltige Sulfonate.

Auch der Ausweg, das entstehende Wasser abzudestillieren, oder, mit reinem $SO_3$ zu sulfieren, ist meist mit Nachteilen verbunden. Er führt zu vermehrter Sulfonbildung, ohne ganz sulfatfreie Sulfonate zu liefern.

In aller Regel ergibt sich also das Problem, das Sulfierungsprodukt, die Sulfonsäure, von überschüssiger Schwefelsäure abzutrennen. Nach den einschlägigen Werken der organischen Chemie, z. B. Houben-Weyl, Bd. IX, Seite 439 bis 444) ist die Isolierung nur dann einfach, wenn die Sulfonsäure die gleiche Anzahl Amin- wie Sulfonsäure-Gruppen enthält und die Verbindung ein schwerlösliches Betain bildet, welches von der verdünnten Schwefelsäure abgesaugt werden kann. Das sind aber Ausnahmen.

Für die übrigen Fälle stehen im technischen Maßstab zwei Standard-Methoden zur Verfügung, von denen jede mit schwerwiegenden Nachteilen behaftet ist.

Bie der ersten, welche allgemein unter der Bezeichnung « Kalken » bekannt ist, neutralisiert man das in Wasser aufgenommene Sulfierungsgemisch mit Ca $(OH)_2$ (gelöschter Kalk, Kalkmilch) oder mit $CaCO_3$ (Kreide oder feingemahlener Kalkstein) und filtriert den ausgefallenen Gips ab. Im Filtrat, welches das Ca-Salz der gebildeten Sulfonsäure enthält, führt man letzteres meist durch Soda oder Glaubersalz ins Na-Salz über und filtriert nochmals vom $CaCO_3$ oder Gips ab, um die erwünschte Lösung des Natriumsalzes zu erhalten. Ein Nachteil dieses Verfahrens ist der hohe Zwangsanfall von Gips und ggf. Kalk, für den in der Regel keine Verwendung gefunden werden kann und der als Abfall die Umwelt belastet. Außerdem ist dieses Verfahren wegen der hohen Flüssigkeitsmengen, die umgewälzt und eingedampft werden müssen, sowie wegen des hohen Zeit- und Arbeitsaufwandes für das zweimalige Filtrieren und das Waschen und schließliche Beseitigen des Filterkuchens recht teuer.

Die zweite Standard-Methode, allgemein unter dem Begriff « Aussalzen » bekannt, beruht darauf, daß man das mit Wasser verdünnte Sulfierungsgemisch mit einem Alkali-Salz (meist NaCl oder $Na_2SO_4$, seltener KCL oder $(NH_4)_2SO_4$) versetzt, um das entsprechende Salz der entstandenen Sulfonsäure auszusalzen. Diese Methode hat den Nachteil, daß mehr oder minder große Mengen Sulfonat in der Mutterlauge verbleiben, oft soviel, daß das Verfahren völlig unbrauchbar ist. Auch ist zu berücksichtigen, daß die isolierten sulfonsauren Salze in der Regel durch das zu ihrer Fällung benutzte anorganische Salz verunreinigt bleiben. Des weiteren sind die Korrosionsprobleme im Falle des Halogenidzusatzes erheblich.

Für beide Standard-Methoden gilt, daß sie nicht ohne das Einbringen von Hilfsstoffen auskommen, die später als solche oder in umgewandelter Form als Abfall erscheinen und die Umwelt belasten.

In der US-PS 37 19 703 sowie in dem übersichtsartikel von Cerny und Hajek in Chemicky Prumysl roc. 29/54 (1979), 410 sowie auch beispielsweise in der JP-A-52-12 147 (Patent Abstracts of Japan, Band 1, Nr. 1 53, 22 Mai 1977, J. 323 C 77) wird beschrieben daß man aromatische Sulfonsäuren aus dem Sulfiergemisch gewinnen kann, indem man dieses mit einem höheren tertiären aliphatischen Amin und einem wasserunlöslichen organischen Lösungsmittel extrahiert, die organische von der wäßrigen Phase abtrennt und das Amin aus der organischen Lösung nach gängigen Methoden isoliert. Das Verfahren ist an sich elegant im Vergleich zu den vorgenannten technischen Verfahren, aber für den großtechnischen Maßstab weniger geeignet, vor allem weil die zu handhabenden großen Lösungsmittelmengen das Reaktionsvolumen unnötig vergrößern und damit die Anlage verteuern, die als Lösungsmittel brauchbaren chlorierten Kohlenwasserstoffe giftig und die unchlorierten feuergefährlich sind und insbesondere, weil der betriebliche Aufwand zur Kontrolle und Konstanthaltung der Zusammensetzung des Amin/Lösungsmittelgemisches und zur vollständigen Entfernung von Lösungsmittelresten aus der Sulfonsäure und der Schwefelsäure zur Vermeidung von Umweltbelastungen erheblich wäre. Cerny und Hajek betonen die entscheidende Bedeutung der Auswahl des richtigen Lösungsmittels für den Erfolg des Verfahrens. Mit der Möglichkeit einer lösungsmittelfreien Verfahrensweise rechneten sie ebenso wenig wie die Erfinder zu dem obengenannten US-Patent. In der DE-C-886 144 wird beschrieben, daß man Sulfonierungsgemische unter Ausschluß von Wasser mit einem organischen Amin behandeln kann, um in der überschüssigen Aminphase gelöste unsulfonierte Anteile abtrennen zu können.

Der Erfindung lag daher die Aufgabe zugrunde, ein möglichst einfaches, wirtschaftliches und umweltfreundliches, für den technischen Maßstab geeignetes Verfahren zu entwickeln.

Die überraschende Entdeckung, daß stöchiometrische Mengen lösungsmittelfreier wasserunlöslicher Amine ohne Emulgierwirkung aus einer Schwefelsäure/Sulfonsäure-Mischung, die im wesentlichen frei von unsulfoniertem Ausgangsmaterial ist, mit hoher Selektivität nur die Sulfonsäure binden, und daß sich das dabei entstehende flüssige Salz (es muß unterhalb der Siedetemperatur der wäßrigen Schwefelsäure flüssig sein, sonst ist es für das erfindungsgemäße Verfahren nicht geeignet. In aller Regel ist es schon bei Raumtemperatur flüssig) auch ohne organisches Lösungsmittel spontan von

der wäßrigen Phase abtrennt, ermöglicht ein neues Verfahren, das als regenerierbaren, also weder die Umwelt noch die Kosten belastenden Hilfsstoff nur das Amin benötigt. Die Schwefelsäure wird dabei nicht in ein Salz übergeführt, sondern fällt als mehr oder weniger konzentrierte (bis 40 %ige) wäßrige Lösung an, die ggf. wirtschaftlich weiterverwendet werden kann. Außerdem ergeben sich wesentliche technische Vorteile durch den Wegfall der obengenannten Nachteile der bekannten Verfahren.

Zur Lösung der Aufgabe wird das anmeldungsgemäße Verfahren folgendermaßen ausgeführt :

Zum Sulfiergemisch wird soviel Wasser gegeben, wie zur Verdünnung der vorhandenen Schwefelsäure auf 20 bis 75, insbesondere 25 bis 50 % nötig ist. Der Trennfaktor bei der späteren Phasentrennung steigt mit zunehmender Verdünnung der Schwefelsäure. Selbstverständlich kann das Verfahren auch bei noch verdünnterer Schwefelsäure angewandt werden. Bei Wiederverwendung der Schwefelsäure ist jedoch eine allzu hohe Verdünnung unzweckmäßig. Die verdünnte Lösung versetzt man mit einer der vorhandenen Sulfonsäuremenge äquivalenten (stöchiometrischen) Menge eines wasserunlöslichen Amins. Nach intensivem Mischen bilden sich zwei Phasen, eine wäßrige, in der Regel untere Schwefelsäurephase und eine sulfatarme Phase des Salzes des organischen Amins mit der Sulfonsäure. Die Schwefelsäure-Phase wird abgetrennt. Die organische Phase wird zweckmäßig mit Wasser, gegebenenfalls mehrmals, gewaschen, um Schwefelsäurereste abzutrennen. Das Waschwasser kann die obere oder untere Phase bilden. Auch die abgetrennte Schwefelsäure-Phase kann zur Erhöhung der Ausbeute noch einmal mit etwas Amin nachgewaschen werden. Schließlich setzt man aus der organischen Phase das Amin mit der äquivalenten Menge der wasserlöslichen Base, deren sulfonsaures Salz hergestellt werden soll, unter Zusatz von Wasser in Freiheit. Man erhält so, unter Wiedergewinnung des wasserunlöslichen Amins, eine konzentrierte wäßrige Lösung des gewünschten Sulfonsäuresalzes. Das Amin steht für einen erneuten Einsatz zur Verfügung. Auch die Waschwässer können beim nächsten Aufarbeitungszyklus wieder eingesetzt werden.

Vorzugsweise wird das beschriebene Verfahren kontinuierlich ausgeführt, wobei die für Stofftrennungen üblichen Apparate, wie Extraktionskolonnen, Mixer-Settler, Scheiblinge oder Trennzentrifugen angewandt werden.

Schematisch kann der Prozeß folgendermaßen dargestellt werden :

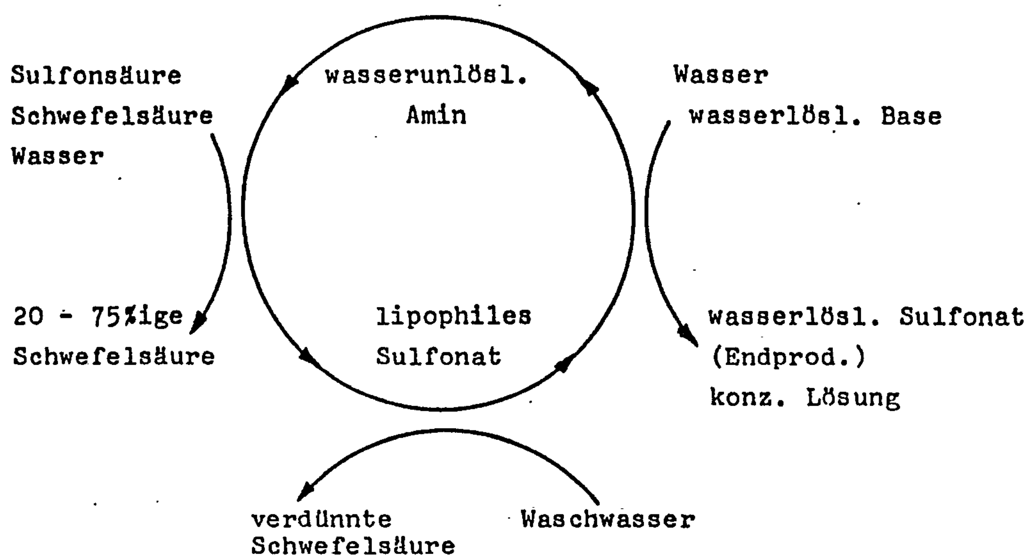


Die Reinheit des Produktes nach dem lösungsmittelfreien Verfahren läßt sich verbessern, wenn man die Alkalifizierung der organischen Phase in zwei Stufen, zuerst bis pH etwa 4, dann nach Phasentrennung mit der restlichen Menge wasserlöslicher Base durchführt. Bis pH 4 geht nämlich das Sulfat bevorzugt in die wäßrige Phase. Man erhält so Produkte mit Sulfat-Gehalten unter 1 %. Der Vorextrakt bis pH 4 kann zur Vermeidung eines geringen Verlustes an Wertprodukt (angestrebtes Endprodukt, also Sulfonat) dem nächsten Ansatz zugegeben werden.

Die Aminverluste betragen bei entsprechend genauer Amindosierung nur Bruchteile eines Prozentes, die Verluste an Sulfonsäure in der Schwefelsäure wenige Prozente. Sie können wie erwähnt durch Nachextraktion der Schwefelsäure mit dem wasserunlöslichen Amin weiter gesenkt werden.

Für die Isolierung in Form ihrer Salze gemäß dem erfindungsgemäßen Verfahren eignen sich z. B. folgende Sulfonsäuren :

Mono-, Di- und Tri-Sulfonsäuren des Benzols, Naphthalins und Anthracens, die eine oder mehrere Alkyl-, Hydroxialkyl-, phenolische Hydroxyl-, gegebenenfalls substituierte, vorzugsweise hydroxyalkylsubstituierte Amingruppen, Carbonyl-, Carboxyl-, Nitril-, Nitro-, Halogen-, Azo-, Alkyl- und/oder Arylsulfon-Gruppen enthalten können.

Desgleichen können Sulfonsäuren heterocyclischer Verbindungen mit den gleichen Substituenten wie oben angeführt eingesetzt werden. Auch Aromaten, die über Äther-, Thio- äther-, Methylen- oder

Sulfon-Brücken zu höhermolekularen Einheiten (Telomeren) verknüpft sind, z. B. wasserlösliche Naphthalin/Formaldehyd/Schwefelsäure-Kondensate, können von synthesebedingter, unerwünschter Schwefelsäure befreit werden.

Die Sulfonsäuren sollen, ebenso wie die Amine, keine emulgierende Wirkung haben. Andernfalls macht die Phasentrennung infolge Emulsionsbildung Schwierigkeiten.

Als wasserunlösliche Amine ohne emulgierende Wirkung, die mit der Sulfonsäure ein genügend lipophiles flüssiges Salz bilden, daß dieses sich spontan von der wäßrigen Phase trennt, kommen wasserunlösliche tertiäre und sekundäre offenkettige verzweigte oder unverzweigte aliphatische Amine mit insgesamt 12 bis 40, vorzugsweise 16 bis 36 Kohlenstoffatomen in Betracht. Als Beispiele seien erwähnt :

Tri-n-octyl-, Tri-2-äthylhexyl-, Methyldioctyl-, Methyldidecyl-, Tridodecyl-, Tributyl-, Di-2-äthylhexyl-, Didecyl-amin.

Sind im Reaktionsgemisch in geringer Menge Neutralsubstanzen, z. B. Diarylsulfonverbindungen, vorhanden, so gehen sie in die organische Phase. Sie können daraus nach üblichen Methoden entfernt werden (z. B. Filtration, Destillation). Das Sulfiergemisch soll jedoch im wesentlichen frei sein von Neutralsubstanzen, insbesondere auch von unsulfonierter Ausgangssubstanz, d. h. : frei von solchen Neutralsubstanz-Mengen, daß diese als Lösungsmittel für das Amin-Sulfonsäure-Salz eine wesentliche Rolle spielen könnten.

Als Basen für die Überführung der Sulfonsäure in die Wasserphase und gleichzeitig in das als Endprodukt gewünschte Salz sind die Hydroxide der Alkali- und Erdalkalimetalle, vor allem Natriumhydroxid, daneben auch die Alkalicarbonate geeignet. Auch mit Ammoniak und wasserlöslichen, aliphatischen, nicht emulgierend wirkenden Aminen läßt sich eine Überführung in die wäßrige Phase erreichen. Solche Amine sind beispielsweise Methyl-, Äthyl-, Propyl-, Butyl-, 2-Hydroxyäthyl-(= Äthanol-), 2-Hydroxypropyl-Amin, wobei die Substituenten jeweils ein-, zwei- oder (außer Äthyl, Propyl und Butyl) dreifach (primäres, sekundäres oder tertiäres Amin) oder auch verschiedene der Substituenten gemischt vorhanden sein können. Triäthylamin und seine höheren Homologen sind allerdings schon zu schwach wasserlöslich. Andererseits ist Monoamylamin noch brauchbar. Trotz ihrer vergleichsweise geringen Basizität sind insbesondere Alkanolamine zur Überführung von Sulfonaten in die wäßrige Phase geeignet.

Wenn bei den Aminen und deren Sulfonaten von der Löslichkeit oder Unlöslichkeit in Wasser bzw. von der zur Trennung von der wäßrigen Phase ausreichenden Lipophilie die Rede ist, so ist dabei stets an eine für praktische Zwecke im Sinne der Erfindung ausreichende Löslichkeit oder Unlöslichkeit bzw. Trennfähigkeit gedacht. Verluste bzw. Verunreinigungen von wenigen Prozent, die auf einer nicht 100 % igen Unlöslichkeit beruhen, können je nach Umständen in Kauf genommen oder durch Wiederholung der Trennoperation minimiert werden.

Der Prozeß ist in einem weiten Temperaturbereich von − 10 bis 130 °C, vorzugsweise bei 20 bis 90 °C durchführbar und kann ohne weiteres den Gegebenheiten des spezifischen Problems angepaßt werden. Ist die organische Phase viskos, so empfiehlt es sich, bei höherer Temperatur zu arbeiten, um die Trennzeit der Phasen zu verkürzen.

Das Amin mit 12 bis 40 C-Atomen sollte möglichst genau stöchiometrisch zur vorhandenen Sulfonsäure eingesetzt werden, da ein Amin-Überschuß Schwefelsäure in die organische Phase einschleppt, wenn es sich um Amine mit mehr als 16 C-Atomen handelt. Bei C-Atom-Zahlen von 12 bis 15 ist überschüssiges Amin in der Schwefelsäure löslich und geht so dem Prozeß verloren. Wenn völlige Sulfatfreiheit des Endproduktes wichtiger ist als eine möglichst hohe Ausbeute, so empfiehlt es sich, das Amin in geringem molaren Unterschuß gegenüber der Sulfonsäure einzusetzen.

Beispiel 4 stellt einen Sonderfall dar : dort enthält die Sulfonsäure ein aromatisch gebundenes Stickstoffatom, das seinerseits ein Mol Schwefelsäure bindet. Deshalb müssen dort zwei Mol Amin pro Mol Sulfonsäure zur Bildung des lipophilen Sulfonates eingesetzt werden. Derartige besondere Verhältnisse müssen selbstverständlich von Fall zu Fall berücksichtigt werden.

Auch die wasserlösliche Base sollte in möglichst genau stöchiometrischer Menge eingesetzt werden, denn ein Unterschuß würde die Ausbeute verschlechtern, und ein Überschuß würde das Endprodukt — insbesondere wenn dieses leicht löslich ist und daher nicht durch Kristallisation isoliert werden kann, sondern entweder in Lösung bleibt oder seine Lösung zur Trockne eingedampft wird — verunreinigen. Mit der stöchiometrischen Menge Alkalihydroxid oder Soda stellt sich ein pH-Wert im Bereich von 8 bis 12, meist 9 bis 11 ein, während er bei Verwendung eines wasserlöslichen Amins niedriger liegt, etwa im Bereich von 5 bis 8.

Das Endprodukt wird entweder in wäßriger Lösung belassen oder durch Eindampfen oder Kristallisation und Dekantieren oder Abfiltrieren gewonnen. Es kann verschiedenster Natur sein, brauchbar beispielsweise als Farbstoff, als Gerbstoff, als Zwischenprodukt bei zahlreichen Synthesen, als Hilfsmittel unter anderem für die Galvano-Industrie, als Dispergiermittel, hydrotrope Verbindung u. a. m.

Ein wesentlicher Vorteil des anmeldungsgemäße Verfahrens gegenüber den bisher technisch ausgeübten Verfahren besteht neben der wirtschaftlichen Wiedergewinnung der Schwefelsäure und dem Entfallen umweltbelastender Abfallstoffe in einer erheblichen Energie- und Arbeitseinsparung : beim Kalken müßte die wäßrige Lösung des Endproduktes vom Caciumsulfat abfiltriert werden. Dazu durfte sie eine gewisse Viskosität, d. h. eine gewisse Konzentration (max. etwa 20 bis 25 %) nicht überschreiten.

Außerdem mußte der Filterkuchen nachgewaschen werden, so daß die Lösung durch die Waschwässer weiter verdünnt wurde. Das Ganze wiederholte sich ggf. noch einmal bei der Überführung des erhaltenen Calciumsalzes der Sulfonsäure in das in der Regel als Endprodukt gewünschte Natriumsalz durch Umsetzung mit Soda oder Glaubersalz. Bei dem erfindungsgemäßen Verfahren ist die Konzentration jedoch nur durch die Löslichkeit des Endproduktes in Wasser begrenzt, d. h. man kann bis zu etwa 50 % ige wäßrige Lösungen erhalten. Die Einsparung an Energie sowie auch Zeit und Arbeitskraft liegt auf der Hand.

Gegenüber dem Verfahren der US-PS 3 719 703 sowie der eingangs genannten tschechischen Literaturstelle hat das anmeldungsgemäße Verfahren u.a. den Vorteil eines kleineren Reaktionsvolumens und damit geringerer Investitionen, der Vermeidung von Vergiftungs- oder Brandgefahren und eines hohen betrieblichen Aufwandes für Kontrolle und Konstanthaltung der Zusammensetzung des Amin/Lösungsmittelgemisches sowie zur Vermeidung von Umweltbelastungen, z. B. durch Lösungsmittel im Abwasser.

Die in den Beispielen genannten Teile und Prozente beziehen sich auf das Gewicht.

## Beispiel 1

Na-m-Nitrobenzolsulfonat

Ein Sulfiergemisch, bestehend aus 328 Teilen 100 %iger Schwefelsäure und 492 Teilen m-Nitrobenzolsulfonsäure, welches als Verunreinigung 0,7 % Dinitrodiphenylsulfon enthielt, wurde unter Kühlung bei 70 bis 82 °C zu einer gut gerührten Mischung aus 588 Teilen Di-2-Äthylhexyl-amin und 450 Teilen Wasser gegeben.

Nach Ende der Zugabe wurde 10 Minuten weitergerührt und 30 Min. absitzen lassen. Die Unterphase, 650 Teile 39,9 %ige wäßrige $H_2SO_4$, welche als Verunreinigung 0,13 % der eingesetzten Aminmenge und ca. 4,6 % m-Nitrobenzolsulfonsäure enthielt, wurde abgetrennt. Nun wurden erneut 250 Teile Wasser zugegeben und 15 Min. bei 60 bis 70 °C gerührt. Nach der Phasentrennung wurden 330 Teile 19,7 %ige Schwefelsäure abgelassen. Diese 2. Waschphase kann jeweils in die 1. Waschung eines neuen Ansatzes eingeschleust werden.

Die organische Phase wurde mit 655 Teilen 10 %iger Natronlauge bei 70 bis 80° auf pH 10 bis 11 gestellt. Das sich bildende zweiphasige System aus dem freigesetzten Amin (welches durch etwas ausgefallenes Di-m-Nitrophenyl-sulfon getrübt war) und wäßriger Lösung von Na-m-Nitrobenzolsulfonat wurde heiß getrennt. Nach Eindampfen der Lösung bis zur Trockne erhielt man 549 Teile hellgelber Kristalle, die noch 4,8 % $Na_2SO_4$ und 0,3 % Wasser enthielten.

Dies entspricht einer Ausbeute von 96 % d. Th.

Das Amin oder die gesamte Mischung konnten durch Filtration leicht von Dinitrodiphenylsulfon befreit werden.

## Beispiel 2

Beispiel 1 wurde bis nach der Waschung mit 250 Teilen Wasser wiederholt.

Dann wurde die Neutralisation jedoch zunächst nur mit 85 Teilen 10 %iger Natronlauge bis pH 5,0 geführt. Dabei wurden 139,7 Teile anorganische Unterphase abgetrennt, welche 27,9 Teile Natriumsulfat (= 5,7 % des Gesamtsulfats) und 46,9 Teile (0,94 % der Gesamtmenge) Na-m-Nitrobenzolsulfonat enthielten.

Die weitere Zugabe von NaOH bis pH 10,5 und Aufarbeitung wurde wie in Beispiel 1 ausgeführt.

Man erhielt 527 Teile hellgelber Kristalle mit einem Sulfatgehalt von 0,7 % entsprechend einer Ausbeute von 95,7 % d. Th.

## Beispiel 3

Na-α-Naphthalinsulfonat

Zu 315 Teilen eines Sulfiergemisches, welches (neben geringen Anteilen Sulfon) 203,4 Teile α-Naphthalinsulfonsäure und 98 Teile Schwefelsäure (100 %ig) enthielt, wurden unter Kühlung auf 60 bis 70 °C 100 Teile Wasser und 235 Teile Di-2-äthylhexylamin gegeben. Nach Ende der Zugabe wurde 15 Min. weitergerührt und absitzen lassen. Abtrennung der Unterphase ergab 164 Teile einer 38,3 %igen, wäßrigen Schwefelsäure, welche noch 0,7 % der α-Naphthalin-Sulfonsäure als Verunreinigung erhielt. In der ersten Trennung fielen 64 % der Gesamtschwefelsäure an.

Die Oberphase wurde erneut mit 150 Teilen Wasser verrührt. Abtrennung ergab 179,1 Teile wäßrige, 14 %ige Schwefelsäure (28 % der Gesamtmenge). Nach Versetzen der org. Phase mit 600 Teilen Wasser wurde mit 435 Teilen 10 %iger Natronlauge auf pH 10,4 gestellt. Es trennten sich bei 65 °C 1 260 Teile wäßrige Lösung ab, welche 217,4 Teile Feststoff mit einem Sulfatgehalt von 5 % enthielten. Das entspricht einer Ausbeute an Na-α-Naphthalinsulfonat von 94,3 %.

Beispiel 4

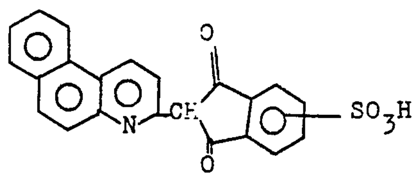

5,6-Benzochinophthalon, sulfiert

Zu 100 Teilen eines Sulfiergemisches, welches 0,57 mMol/g obiger Sulfonsäure enthielt, wurden bei 80 °C 500 Teile Wasser und 43 Teile Ditridecylamin gegeben. Dies ist, berechnet auf die Sulfonsäure, die doppelte molare Menge. Diese ist hier erforderlich, weil der Chinolin-Stickstoff ein Mol Schwefelsäure als Hydrogensulfat bindet.

Nach Abtrennung von 551 Teilen einer wäßrigen Lösung, die 94 % der vorhandenen Schwefelsäure enthielt, wurde die organische Phase mit 114 Teilen 4 %iger Natronlauge behandelt, um weitere 4 % Schwefelsäure zu extrahieren.

Schließlich wurde die organische Phase, bestehend aus einem Gemisch von Ditridecylammoniumsulfonat und freiem Ditridecylamin, mit 10,8 Teilen Triisopropanolamin und 33 Teilen Wasser extrahiert. Der Farbstoff obiger Natur wanderte als Triisopropanolamin-Salz vollständig in die wäßrige Phase. Diese wurde abgetrennt und zur Trockne eingedampft.

Das Ditridecylamin wurde vollständig wiedergewonnen.

Beispiel 5

Na-2-nitro-5-chlor-p-toluolsulfonat

Zu 100 Teilen eines Reaktionsgemisches, welches außer Wasser 468,1 Teile Chlornitrotoluolsulfonsäure und 372,4 Teile Schwefelsäure enthielt, wurden unter Kühlung auf 70 °C 850 Teile Wasser und 431, 4 Teile Di-2-ethyl-hexyl-amin gegeben. Nach Ende der Zugabe wurde weitere 10 Minuten intensiv gerührt und absitzen gelassen. Die abgetrennte wäßrige Unterphase von insgesamt 1 281,4 Teilen enthielt 364,7 Teile Schwefelsäure. Durch high pressure liquid chromatography (HPLC) war in einer Probe keine Chlornitrotoluolsulfonsäure mehr nachzuweisen. Bei der ersten Abtrennung fielen 98,2 % der Schwefelsäure an.

Die Oberphase wurde mit 970 Teilen 8 %iger Natronlauge unter gutem Rühren bis auf Ph 9,7 bei 50-60° neutralisiert. Es schieden sich 1 506 Teile einer wäßrigen Lösung ab, welche 34,4 % Feststoff, davon 0,64 % Natriumsulfat, enthielt. Dies entspricht einer Ausbeute von über 99,5 % an Na-2-nitro-5-chlor-p-toluolsulfonsäure bei einem Natriumsulfat-Gehalt von 1,8 %. Der Amin-Verlust betrug weniger als 0,5 %.

Beispiel 6

Na-Salz eines Naphthalin-Formaldehyd-Schwefelsäure-Kondensationsproduktes

Die Herstellung des Kondensationsproduktes wurde folgendermaßen durchgeführt :

100,0 Tl. Naphthalin wurden mit 115,0 Tl. 98 %iger Schwefelsäure 4 Stdn. auf 150 bis 155 °C gehalten. Zur Mischung der vorwiegend das ß-Isomere enthaltenden Sulfonsäuren wurden nun unter Kühlung auf 100 bis 110 °C 55 Tl. 30 %iger wäßriger Formaldehyd und 55 Teile Wasser gegeben. Nach weiteren 6 Stdn. bei 100° wurde mit 300 Tln. Wasser verdünnt.

Die Analyse dieser Lösung ergab einen Gehalt von 1,005 mVal/g Schwefelsäure und — als Differenz der Gesamtsäure und des Sulfats bestimmt — einen Gehalt von 1,260 mVal/g Sulfonsäure.

Zu 600 Tln. dieser Lösung wurden bei 80 bis 90 °C unter gutem Rühren 140,1 Tl. Tributylamin gegeben. Nach 15-minütigem Rühren wurde die Mischung 10 Minuten zur Ausbildung der Trennphase belassen. Als Oberphase wurden 403 Teile einer 5,93 %igen Schwefelsäure (81,0 % der vorhandenen Menge) abgetrennt. In der Säurephase befanden sich 0,52 % der Sulfonsäuren. Waschung mit weiteren 300 Teilen Wasser ergab nochmals 306 Teile 1,65 %iger Schwefelsäure, entsprechend 17,1 % der vorhandenen Menge.

Neutralisation mit NaOH bis pH 10,5 und nachfolgende Einengung der abgetrennten Wasserphase ergab 182 Teile des Na-Salzes mit einem Sulfat-Gehalt von 0,47 %. Die Ausbeute, bestimmt aus der ursprünglichen Menge abzüglich der Verluste im ersten Waschwasser, betrug 99,5 %.

**Anspruch**

Verfahren zur Abtrennung flüssiger wasserlöslicher Aminsalze von aromatischen Sulfonsäuren aus

Sulfiergemischen, dadurch gekennzeichnet, daß man

a) das Sulfiergemisch, das im wesentlichen frei von unsulfoniertem Ausgangsmaterial und anderen Neutralsubstanzen ist, mit Wasser auf eine Schwefelsäurekonzentration von 20 bis 75 % verdünnt,

b) das verdünnte Gemisch mit einer der Sulfonsäure äquivalenten Menge eines nicht emulgierend wirkenden, lösungsmittelfreien, wasserunlösichen tertiären oder sekundären offenkettigen verzweigten oder unverzweigten aliphatischen Amins mit insgesamt 12 bis 40 Kohlenstoffatomen, das mit der Sulfonsäure ein genügend lipophiles flüssiges Salz bildet, daß es sich spontan von der wäßrigen Phase trennt, vermischt,

c) die zwei entstehenden Phasen verschiedener Dichte trennt,

d) die aus dem lipophilen Ammoniumsalz der aromatischen Sulfonsäure bestehende Phase, ggf. nach Waschen mit Wasser, mit der stöchiometrischen Menge einer wasserlöslichen Base, deren sulfonsaueres Salz hergestellt werden soll, und Wasser versetzt,

e) wiederum die zwei entstehenden Phasen trennt und das Salz in der wäßrigen Lösung beläßt oder

f) es durch Eindampfen oder Kristallisation und Dekantieren oder Filtrieren isoliert.

**Claim**

A process for isolating liquid water-soluble salts of aromatic sulfonic acids from sulfonation mixtures, wherein

a) the sulfonation mixture which is esentially free of unsulfonated starting material and other neutral substances is diluted with water to a sulfuric acid concentration of from 20 to 75 % by weight,

b) the diluted mixture is mixed with an amount, equivalent to the sulfonic acid, of a solvent-free, water-insoluble, open-chain, branched or linear, tertiary or secondary aliphatic amine of a total of 12 to 40 carbon atoms, which does not have an emulsifying action and which forms, with the sulfonic acid, a liquid salt which is sufficiently lipophilic that it separates spontaneously from the aqueous phase,

c) the two resulting phases, which are of different density, are separated,

d) the phase which consists of the lipophilic ammonium salt of the aromatic sulfonic acid is mixed, if necessary after having been washed with water, with the stoichiometric amount of a water-soluble base whose sulfonate is to be prepared, and with water,

e) the two resulting phases are again separated and the salt is left in the aqueous solution or

f) the salt is isolated by evaporation or by crystallization and decanting or filtering.

**Revendication**

Procédé de séparation de sels aminés liquides, solubles dans l'eau, d'acides sulfoniques aromatiques, présents dans des mélanges sulfonés, caractérisé par le fait qu'

a) on dilue le mélange sulfoné, qui est, pour l'essentiel, exempt de matériau de départ non sulfoné et d'autres substances neutres, avec de l'eau, à une concentration en acide sulfurique de 20 à 75 %,

b) on mélange le mélange dilué avec une quantité, équivalente à l'acide sulfonique, d'une amine 1, 2, 3, 4, 5, 6 et n'agissant pas de manière émulsifiante, aliphatique, ramifiée ou non, à chaîne ouverte, tertiaire ou secondaire, insoluble dans l'eau, exempte de solvant, ayant au total 12 à 40 atomes de carbone et qui forme avec l'acide sulfonique un sel liquide, suffisamment lipophile pour se séparer spontanément de la phase aqueuse,

c) on sépare les deux phases produites de densités différentes,

d) on fait réagir la phase composée du sel d'ammonium lipophile de l'acide sulfonique aromatique, éventuellement après lavage avec de l'eau, avec la quantité stoechiométrique d'une base soluble dans l'eau, dont le sel sulfonique doit être préparé, et de l'eau,

e) on sépare à nouveau les deux phases produites et on abandonne le sel dans la solution aqueuse ou

f) on l'isole par évaporation ou cristallisation et décantation ou filtration.